# EUROPÄISCHE PATENTSCHRIFT

(11) **EP 2 114 348 B1**
(45) Veröffentlichungstag und Bekanntmachung des Hinweises auf die Patenterteilung: **16.05.2012**
(21) Anmeldenummer: 08734354.7
(22) Anmeldetag: 06.03.2008
(51) Int. Cl.: A61K 6/027, A61K 6/06

(54) **VERBLENDKERAMIK FÜR DENTALE RESTAURATIONEN AUS YTTRIUMSTABILISIERTEM ZIRKONIUMDIOXID**
VENEERING CERAMIC FOR DENTAL RESTORATIONS MADE OF YTTRIUM-STABILIZED ZIRCONIUM DIOXIDE, AND METHOD FOR VENEERING DENTAL RESTORATIONS MADE OF YTTRIUM-STABILIZED ZIRCONIUM DIOXIDE
CÉRAMIQUE POUR INCRUSTATIONS VESTIBULAIRES POUR DES RESTAURATIONS DENTAIRES EN DIOXYDE DE ZIRCONIUM STABILISÉ À L'YTTRIUM ET PROCÉDÉ DE FIXATION D'INCRUSTATIONS VESTIBULAIRES SUR DES RESTAURATIONS DENTAIRES EN DIOXYDE DE ZIRCONIUM STABILISÉ À L'YTTRIUM

(30) Priorität: 06.03.2007 DE 102007011337
(43) Veröffentlichungstag der Anmeldung: 11.11.2009
(73) Patentinhaber: Ivoclar Vivadent AG, 9494 Schaan (LI)
(72) Erfinder: JOHANNES, Martina, 07629 Hermsdorf (DE); EHRT, Roland, 07747 Jena (DE)
(74) Vertreter: UEXKÜLL & STOLBERG
(86) Internationale Anmeldenummer: PCT/DE2008/000405
(87) Internationale Veröffentlichungsnummer: WO 2008/106958

(56) Entgegenhaltungen:
- EP-A1- 1 505 041
- WO-A1-03/035014
- WO-A2-02/47616
- US-A- 4 798 536
- US-A- 5 217 375

## Beschreibung

Die Erfindung betrifft Verblendkeramiken für dentale Restaurationen, wobei die Gerüstkeramik aus yttriumstabilisiertem Zirkoniumdioxid besteht.

Yttriumstabilisiertes Zirkoniumdioxid ist ein Hochleistungswerkstoff mit extrem hoher Festigkeit, der in der restaurativen Zahnmedizin in wachsendem Maße für Gerüstkeramiken für Kronen, Inlays sowie Brücken zum Einsatz kommt. Die Feinanpassung an die Vielfalt der natürlichen Zähne erfordert die Anwendung von Verblendkeramiken. In der Belastbarkeit der restaurierten Zähne bildeten bisher die Verblendkeramiken eine Schwachstelle.

Verblendkeramiken sollten eine gute Modellierung ermöglichen, in der Farbgestaltung den benachbarten Zähnen angepasst sein, eine hohe chemische Beständigkeit aufweisen, nach einer gezielten Temperaturbehandlung selbst. über eine hohe Biegebruchfestigkeit verfügen und sich durch eine intensive Haftung mit der Gerüstkeramik auszeichnen.

Als Ausgangsstoffe bei der Herstellung der Verblendkeramiken kommen in der Regel Pulver bzw. Pasten zur Anwendung. Die Eigenschaften der Verblendkeramik werden sowohl von den chemischen und kristallographischen Merkmalen als auch der Körnung der Ausgangsmaterialien bestimmt.

Leucithaltige Dentalkeramiken werden entsprechend Patent US 4,798,536 A über die Glasschmelze gefertigt. Der Leucitgehalt liegt im Bereich von 35 bis 60 Gew %. Der hohe Ausdehnungskoeffizient der leucithalfigen Dentalkeramik von 13 bis 15 x 10⁻⁶/K wird für das Verblenden von Metallkronen genutzt. Die Biegebruchfestigkeit der Verblendkeramik mit Leucitkristallen liegt bei 80 MPa.

Im Patent US 4,189,325 A wird für den restaurativen Zahnersatz die Nutzung von Lithiumdisilicat vorgeschlagen. Dabei wird sich auf das Stoffsystem Li₂O-CaO-Al₂O₃-SiO₂ konzentriert. Zur Unterstützung der Kristallisation werden die Keimbildner Nb₂O₅ und Pt zugesetzt.

Mit dem Patent US 4,515,634 A wird vorgeschlagen, im Grundsystem Li₂O-CaO-Al₂O₃-SiO₂ zur Verbesserung der Keimbildung und Kristallisation den Keimbildner P₂O₅ zuzusetzen.

Die Offenlegungsschrift DE 197 50 794 A1 beschreibt den Einsatz von Lithiumdisilicat - Glaskeramiken für die Nutzung im Heißpressverfahren. Es hat sich jedoch gezeigt, dass bei der Anwendung dieses Verfahrens die Kantenfestigkeit am restaurierten Zahn nicht ausreichend ist und bei der Nachbearbeitung ein hoher Werkzeugverschleiß eintritt.

Mit der DE 103 36 913 A1 wird vorgeschlagen, den zu restaurierenden Zahn in zwei Stufen zu fertigen. In der ersten Stufe wird Lithiummetasilicat zur Kristallisation gebracht, das durch maschinelle Verarbeitung zu dentalen Produkten verarbeitet wird. Mit einer zweiten Temperaturbehandlung wird das Lithiummetasilicat in das festere Lithiumdisilicat überführt. Damit besteht der gesamte restaurierte Zahn aus Glaskeramik mit Lithiumdisilicatkristallen.

In der Patentschrift DE 196 47 739 C2 wird eine sinterbare Lithiumdisilicat-Glaskeramik sowie Glas beschrieben. Das Ausgangsmaterial wird zu Rohlingen gesintert. Diese Rohlinge werden bei 700 °C bis 1200 °C zu dentalen Produkten gepresst. Bei der plastischen Verformung weist die beschriebene Lithiumdisilicat-Glaskeramik nur eine geringe Reaktion mit der benachbarten Einbettmasse auf.

Auf der Grundlage von yttriumstabilisiertem Zirkoniumdioxid wird in der EP 1 235 532 A1 ein Verfahren zur Herstellung von hochfestem keramischen Zahnersatz beschrieben. Die nach diesem Verfahren gefertigten Gerüstkeramiken weisen 4-Punkt Biegebruchfestigkeiten von größer 1200 MPa auf.

Der Erfindung liegt die Aufgabe zugrunde, eine Möglichkeit für eine transluzente Verblendkeramik mit hoher Biegebruchfestigkeit zu schaffen, wobei die Verblendkeramik ferner eine sehr gute Haftung zur Gerüstkeramik aus yttriumstabilisiertem Zirkoniumdioxid aufweisen soll.

Erfindungsgemäß wird diese Aufgabe bei einer Verblendkeramik von aus yttriumstabilisiertem Zirkoniumdioxid hergestellten dentalen Restaurationen dadurch gelöst, dass sie durch die folgenden Komponenten hergestellt wird:

| | | |
|---|---|---|
| a) | SiO₂ | 58,0 - 74,0 Gew. % |
| b) | Al₂O₃ | 4,0 - 19,0 Gew. % |
| c) | Li₂O | 5,0-17,0 Gew. % |
| d) | Na₂O | 4,0 -12,0 Gew. % |
| e) | ZrO₂ | 0,5 - 6,0 Gew. % |

Von Vorteil kann es sein, wenn neben dem Keimbildner ZrO₂ ein weiterer Keimbildner, z.B. TiO₂ in den Grenzen von 0,2 bis 8,0 Gew. % beigefügt wird.

Die Verblendkeramik kommt als gepulvertes Ausgangsglas mit kristallinen Zusätzen bzw. ohne gesonderte kristalline Zusätze zur Anwendung und wird mittels definiertem Temperaturprogramm im Bereich zwischen 800 und 940°C auf dentale Produkte aus yttriumstabilisiertem Zirkoniumdioxid aufgesintert und kontrolliert kristallisiert.

Es hat sich überraschenderweise gezeigt, dass mit spezifischen Glaskeramiken und einem definierten Temperaturprogramm eine sehr hohe Haftfestigkeit auf dentalen Produkten aus yttriumstabilisiertem Zirkoniumdioxid erreicht wird. Die Verblendkeramik ist dabei transluzent und hat eine sehr gute chemische Beständigkeit. Die Hauptkristallphase der Glaskeramik besteht aus Lithiumdisilicat.

Als Ausgangsprodukt kann die Verblendkeramik außer den gepulverten Ausgangsgläsern der Glaskeramik auch gepulverte Kristalle enthalten. Über eine definierte Temperaturbehandlung durchläuft die gepulverte Verblendkeramik die Prozesse Keimbildung, Sintern und Verschmelzen mit dem yttriumstabilisierten Zirkoniumdioxid sowie Kristallisation unter Bildung von Mikrokristallen.

Es ist ebenfalls bevorzugt, dass den Ausgangsgläsern gepulvertes Lithiumdisilicat. zugesetzt wird. Das Lithiumdisilicat kann über eine Festkörperreaktion hergestellt werden.

Die Zugabe von TiO₂ unterstützt den Prozess der Keimbildung sowie der Kristallisation von Lithiumdisilicat. Die Verblendkeramik wird dann vorteilhafterweise aus einer Mischung gebildet, die die folgenden Komponenten enthält:

| | | |
|---|---|---|
| a) | SiO₂ | 58,0 - 72,0 Gew. % |
| b) | Al₂O₃ | 4,0-18,0 Gew. % |
| c) | Li₂O | 5,0 -17,0 Gew. % |
| d) | Na₂O | 4,0 - 11,0 Gew. % |
| e) | ZrO₂ | 0,5 - 5,5 Gew. % |
| f) | TiO₂ | 0,2 - 8,0 Gew. % |

Für die gesteuerte Kristallisation der Verblendkeramik auf Basis von Lithiumsilicatmaterialien kommt als Keimbildner Zirkoniumdioxid bzw. ein Gemisch aus Zirkoniumdioxid und Titandioxid zum Einsatz. Die Zugabe von Titandioxid begünstigt die Umwandlung von Lithiummetasilicat in Lithiumdisilicat.

Die Verblendkeramik ist ebenfalls bevorzugt, wenn neben Lithiumdisilicat auch Lithium Titanium Oxid Silicat Li₂TiOSiO₄ ,Lithiumalumosilicat (β - Spodumen) sowie geringere Mengen Lithiummetasilicat auskristallisieren.

Die Verblendkeramik kann auch so gestaltet sein, dass der kristalline Anteil unter 40 % liegt. Dabei wird die Verblendkeramik dünn aufgetragen. In diesem Fall dient die Verblendkeramik der Farbanpassung und verleiht der dentalen Gerüstkeramik einen besonderen ästhetischen Glanz. Durch gezielte Druckspannungen kann die Festigkeit der Verblendkeramik weiter erhöht werden.

Als färbende bzw. fluoreszierende Zusätze können die Oxide der Elemente Ce, Fe, Mn, Sn, V, Cr, In sowie der Seltenen Erden Pr, Nd, Sm, Eu, Tb, Dy und Er zum Einsatz kommen.

Zur Modifizierung der Technologie können unabhängig voneinander die Zusätze La₂O₃, B₂O₃, P₂O₅, CaO, MgO, ZnO und Fluorid zugegeben werden, wobei die Konzentration maximal im Bereich bis zu 4,0 Gew % liegt.

Für die Herstellung der Verblendkeramik erfolgt die Keimbildung im Temperaturbereich von 500 bis 680 °C und das Verschmelzen und Kristallisieren im Temperaturbereich von 800 bis 940 °C. Der Keimbildungs- und Kristallisationsprozess kann unterbrochen werden, indem die Verblendkeramik zwischen Keimbildung und Kristallisation auf Zimmertemperatur abgekühlt, gelagert und danach auf die Kristallisationstemperatur erhitzt wird.

Die Bestimmung der Haftfestigkeit zwischen dem yttriumstabilisiertem Zirkoniumdioxid und der Verblendkeramik erfolgt im Biegeversuch. Dazu wird die gepulverte Verblendkeramik auf die Stirnfläche von zwei Rundstäben aus Zirkoniumdioxid aufgebracht und der definierten Temperaturbehandlung unterzogen. Die Bestimmung der Haftfestigkeit erfolgt im Dreipunktbiegeversuch.

Bevorzugt werden Verblendkeramiken, deren Haftfestigkeit zum Zirkoniumdioxid mindestens 150 MPa beträgt.

Die Erfindung soll nachstehend anhand von Ausführungsbeispielen näher erläutert werden. Die Zeichnungen zeigen:
- Fig. 1: Röntgendiffraktogramm (XRD) nach der Festkörperreaktion von Lithiumoxid und Siliziurridioxid (vier Stunden bei 940 °C),
- fig. 2: einen typischen Temperaturverlauf zur Herstellung der Verblendkeramik und
- Fig. 3: XRD einer erfindungsgemäßen Verblendkeramik.

Als Ausführungsbeispiele der erfindungsgemäßen Verblendkeramiken sind in der Tabelle 1 zwölf Synthesen ausgewiesen.

**Tabelle 1**

| Beispiele | 1 | 2 | 3 | 4 | 5 | 6 | 7 | 8 | 9 | 10 | 11 | 12 |
|---|---|---|---|---|---|---|---|---|---|---|---|---|
| | | | | | | | | | | | | |
| SiO₂ | 71,0 | 71,1 | 62,0 | 0,5 | 69,7 | 61,2 | 70,5 | 70,5 | 69,6 | 69,6 | 58,9 | 60,5 |
| Al₂O3 | 9,0 | 4,9 | 17,9 | 8,9 | 4,8 | 17,7 | 4,9 | 8,9 | 8,8 | 8,8 | 17;0 | 17,5 |
| Li₂O | 12,6 | 14,9 | 5,3 | 12,5 | 14,6 | 5,2 | 14, 8 | 12,5 | 12,4 | 12,4 | 5,0 | 5,2 |
| Na₂O | 5,4 | 3,0 | 10,9 | 5,4 | 2,9 | 10,8 | 3,0 | 5,4 | 5,3 | 5,3 | 10,4 | 10,5 |
| TiO₂ | | 5,0 | | | 4,9 | 1,2 | 5,0 | | | | 5,0 | 2,5 |
| ZrO₂ | 2,0 | 1,1 | 3,9 | 2,0 | 1,1 | 3,9 | 1,1 | 2,0 | 2,0 | 2,0 | 3,7 | 3,8 |
| CaF₂ | | | | 0,7 | | | 0,7 | | | | | |
| CaO | | | | | 0,6 | | | | | | | |
| MgF₂ | | | | | | | | 0,7 | | | | |
| BaF₂ | | | | | | | | | | 1,9 | | |
| BaO | | | | | | | | | 1,1 | | | |
| P₂O₅ | | | | | 1,4 | | | | 0,8 | | | |
| | | | | | | | | | | | | |
| Summe | 100 | 100 | 100 | 100 | 100 | 100 | 100 | 100 | 100 | 100 | 100 | 100 |

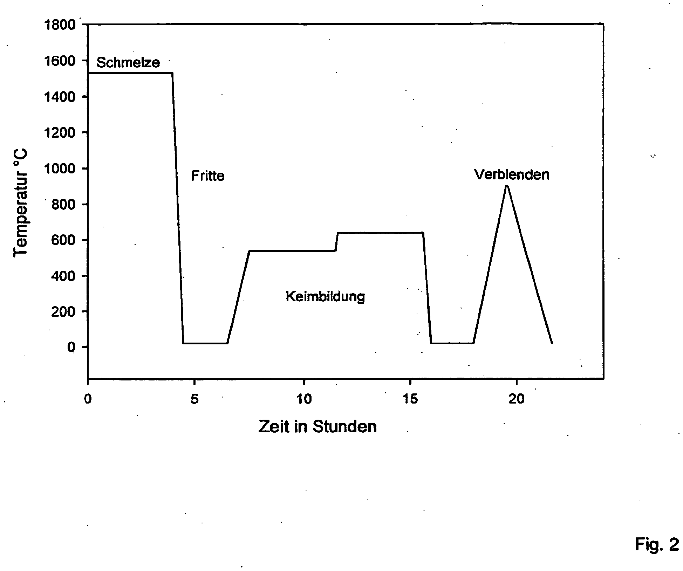

Die Ausgangsgläser wurden in Platin bzw. Platin - Rhodium - Tiegeln bei einer Temperatur von 1530 °C geschmolzen und zur Herstellung einer Fritte in Wasser gegossen (Figur 2).

Zur Unterstützung der gesteuerten Kristallisation werden die gefritteten Ausgangsgläser ca. vier Stunden bei 580 ± 100 °C getempert und nach dem Abkühlen pulverisiert. Die zum Einsatz gebrachte Korngröße liegt im Bereich von 0,6 µm bis 20 µm.

Den Ausgangsgläsern kann gepulvertes Lithiumdisilicat zugesetzt werden. Dabei wird das Lithiumdisilicat über eine Festkörperreaktion hergestellt.

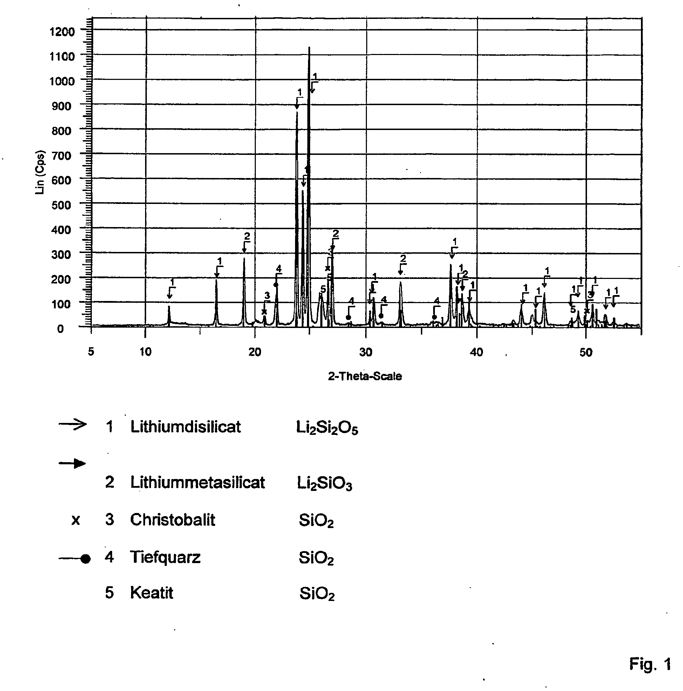

Figur 1 zeigt das Röntgendiffraktogramm (XRD) des über die Festkörperreaktion hergestellten Lithiumdisilicates.

Die angefeuchteten Ausgangsmaterialien werden als Verblendkeramik auf die dentale Gerüstkeramik aus yttriumstabilisiertem Zirkoniumdioxid aufgetragen und bei 890 ± 50 °C verschmolzen und gesteuert kristallisiert.

In Figur 2 ist der typische Temperaturverlauf während des Herstellungsprozesses der Verblendkeramik dargestellt.

Alle zwölf Beispiele der in Tabelle 1 ausgewiesenen Verblendkeramiken sind transluzent.

Sowohl die optische Wirkung als auch die mechanische Widerstandskraft der Verblendkeramik werden sowohl durch das Gefüge der Verblendkeramik als auch von der Wechselwirkung von Verblendkeramik und Gerüstkeramik beeinflusst.

Der Ausdehnungskoeffizient (α) von der Verblendkeramik und der Gerüstkeramik aus yttriumstabilisiertem Zirkoniumdioxid (TZ3Y) muss aufeinander abgestimmt sein. Ausgehend von Beispielen der Tabelle 1 werden in der Tabelle 2 die Ausdehnungskoeffizienten (a) der Verblendkeramiken dokumentiert und mit yttriumstabilisiertem Zirkoniumdioxid verglichen.

**Tabelle 2**

| Beispiele | 1 | 2 | 3 | 6 | 11 | ZrO₂ |
|---|---|---|---|---|---|---|
| | | | | | | |
| α_{50-300°C} x 10⁻⁶/K | 8,9 | 8,7 | 8,9 | 8,9 | 8,7 | 9,6 |
| | | | | | | |
| α_{50-500°C} x 10⁻⁶/K | 9,8 | 9,8 | 9,8 | 9,6 | 9,3 | 9.8 |

Die Haftfestigkeit zwischen Gerüstkeramik aus yttriumstablisiertem Zirkoniumdioxid und der Verblendkeramik wurde im Dreipunkt - Biegeversuch bestimmt. Dazu wurde das Pulver der Verblendkeramik zwischen zwei zylindrische Probenkörper aus Zirkoniumdioxid aufgebracht und einer Temperaturbehandlung entsprechend Figur 2 unterzogen.

Für ausgewählte Proben ist in Tabelle 3 die Haftfestigkeit ausgewiesen, wobei σ = Haftfestigkeit in MPa nach Dreipunktbiegeversuch und m = Weibull Parameter sind.

**Tabelle 3**

| Beispiele | 1 | 2 | 3 | 12 |
|---|---|---|---|---|
| | | | | |
| σ MPa | 162,1 | 183,1 | 173,4 | 172,4 |
| m | 7,6 | 13,2 | 3,5 | 4,4 |

Bei den erfindungsgemäßen Verblendkeramiken mit hoher Haftfestigkeit sind in Abhängigkeit von der Zusammensetzung und der Temperaturbehandlung unterschiedliche Keimbildungs- und Kristallisationsabläufe möglich.

Bezogen auf Tabelle 1 und einer Temperatur von 890 ± 50 °C kristallisieren die Beispiele der Verblendkeramiken 1, 4, 8, 9 und 10 zu den Kristallphasen Lithiumsilicat und Zirkoniumdioxid. Dabei dient das Zirkoniumdioxid als Keimbildner.

Die Kristallisation des Lithiumsilicates erfolgt in zwei zeitlichen Etappen. Zuerst bildet sich Lithiummetasilicat Li₂SiO₃ und durch die anschließende Reaktion mit der umgebenden Silicatphase wandelt sich Lithiummetasilicat in Lithiumdisilicat Li₂Si₂O₅ um.

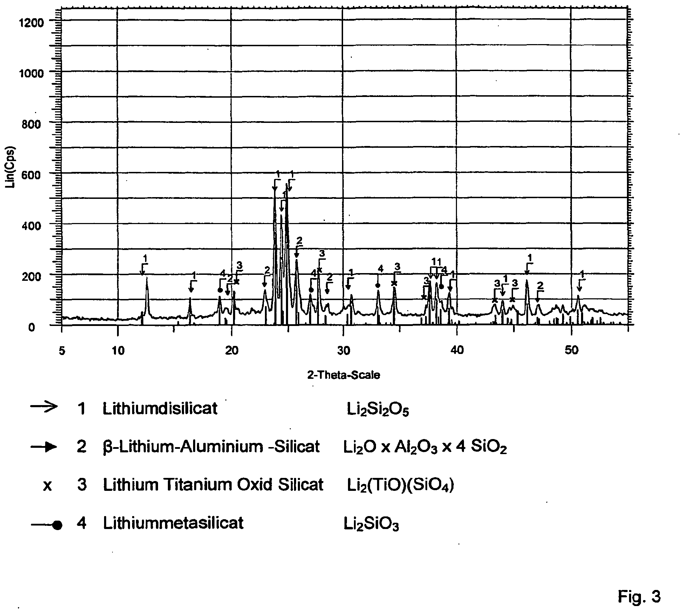

Bezogen auf Tabelle 1 und einer Temperatur von 890 ± 50 °C kristallisieren die Beispiele der Verblendkeramiken 2, 5 und 7 zu den Kristallphasen Lithiumdisilicat, β - Spodumen, Lithium Titanium Oxide Silicate Li₂ (TiO)(SiO₄) und Lithiummetasilicat. Durch den Zusatz von Titandioxid wird die Kristallisation von Lithiumdisilicat Li₂Si₂O₅ beschleunigt. In Figur 3 wird die XRD - Aufnahme ausgewiesen.

## Patentansprüche

1. Verblendkeramik für dentale Restaurationen aus yttriumstabilisiertem Zirkoniumdioxid, **dadurch gekennzeichnet, dass** sie die folgenden Komponenten enthält:
| | | |
|---|---|---|
| a) | SiO₂ | 58,0 - 74,0 Gew. % |
| b) | Al₂O₃ | 4,0-19,0 Gew. % |
| c) | Li₂O | 5,0 - 17,0 Gew. % |
| d) | Na₂O | 4,0-12,0 Gew. % |
| e) | ZrO₂ | 0,5 - 6,0 Gew. % |

2. Ausgangsglas für Verblendkeramjken für dentale Restaurationen aus vttriumstabilisiertern Zirkoniumdioxid, **dadurch gekennzeichnet, dass** das Ausgangsglas die folgenden Komponenten enthält:
| | | |
|---|---|---|
| a) | SiO₂ | 58.0 - 74,0 Gew. % |
| b) | Al₂O₃ | 4.0-19.0 Gew. % |
| c) | Li₂O | 5.0-17,0 Gew. % |
| d) | Na₂O | 4.0-12.0 Gew. % |
| e) | ZrO₂ | 0.5 - 6,0 Gew. % |

3. Ausgangsglas gemäß Anspruch 2, **dadurch gekennzeichnet, dass** es zusätzlich gepulverte Kristalle, vorzugsweise gepulvertes Lithiumdisilicat, enthält.

4. Verblendkeramik gemäß Anspruch 1 oder Ausgangsglas gemäß Anspruch 2 oder 3, **dadurch gekennzeichnet, dass** neben ZrO₂ als weiterer Keimbildner 0,2 bis 8,0 Gew. % FiO₂ enthalten sind.

5. Verblendkeramik gemäß Anspruch 1 oder 4 oder Ausgangsglas gemäß einem der Ansprüche 2. 3 oder 4, **dadurch gekennzeichnet, dass** die folgenden Komponenten enthalten sind:
| | | |
|---|---|---|
| a) | SiO₂ | 58,0 - 72,0 Gew. % |
| b) | Al₂O₃ | 4,0 - 18,0 Gew. % |
| c) | Li₂O | 5,0 - 17,0 Gew. % |
| d) | Na₂O | 4,0 - 11,0 Gew. % |
| e) | ZrO₂ | 0,5 - 5,5 Gew. % |
| f) | TiO₂ | 0,2 - 8,0 Gew. % |

6. Verblendkeramik Gemäß einem der Ansprüche 1. 4 oder 5 oder Ausgangsglas gemäß einem der Ansprüche 2 bis 5, **dadurch Gekennzeichnet, dass** unabhängig voneinander die Zusätze La₂O₃, B₂O₃, P₂O₅, CaO, MgO, ZnO und Fluorid enthalten sind, wobei die Konzentration maximal im Bereich bis zu 4.0 Gew. % liegt.

7. Verblendkeramik gemäß Anspruch 6 oder Ausganosglas gemäß Anspruch 6, **dadurch gekennzeichnet, dass** die folgenden Komponenten enthalten sind:
| | | |
|---|---|---|
| a) | La₂O₃ | 1,0 - 4,0 Gew. % |
| b) | B₂O₃ | 0,0 - 2,0 Gew. % |
| c) | MgO | 0,0 - 2,0 Gew. % |
| d) | CaO | 0,0 - 2,0 Gew. % |
| e) | ZnO | 0,0 - 2,0 Gew. % |
| f) | BaO | 0,0 - 1,0 Gew. % |
| g) | P₂O₅ | 0,0 - 2,0 Gew. % |
| h) | Fluorid | 0,0 - 3,0 Gew. % |

8. Verblendkeramik gemäß einem der Ansprüche 1 oder 4 bis 7 oder Ausgangsglas gemäß einem der Ansprüche 2 bis 7 , **dadurch gekennzeichnet, dass** Oxide der färbenden bzw. fluoreszierenden Zusätze von den Elementen Ce, Fe, Mn, Sn, V, Cr, In sowie der Seltenen Erden Pr, Nd, Sm, Eu, Tb, Dy und Er einzeln oder in Kombination enthalten sind.

9. Verblendkeramik nach Anspruch 1 oder 5, **dadurch gekennzeichnet, dass** zur Unterdrückung der Kristallisation von Hochquarzmischkristallen weitere Alkalioxide, enthalten sind.

10. Verblendkeramik nach einem der Ansprüche 1 oder 4 bis 9, **dadurch gekennzeichnet, dass** sie neben Lithiumdisilicat die Kristallphasen Lithium Titanium Oxid Silicat Li₂ TiOSiO₄, Lithiumalumosilicat (β-Spodumen) und geringere Mengen Lithiummetasilicat enthält.

11. Ausgangsglas für Verblendkeramiken für dentale Restaurationen aus yttriumstabilisiertem Zirkoniumdioxid, **dadurch gekennzeichnet, dass** das Ausgangsglas
| | | |
|---|---|---|
| 71,1 | Gew.-% | SiO₂, |
| 4,9 | Gew.-% | Al₂O₃, |
| 14,9 | Gew.-% | Li₂O, |
| 3,0 | Gew.-% | Na₂O, |
| 5.0 | Gew.-% | -TiO₂ und |
| 1.1 | Gew.-% | ZrO₂ |
enthält.

12. Ausgangsglas für Verblendkeramiken für dentale Restaurationen aus yttriumstabilisiertem Zirkoniumdioxid, **dadurch gekennzeichnet, dass** das Ausgangsglas
| | | |
|---|---|---|
| 69,7 | Gew.-% | SiO₂, |
| 4.8 | Gew.-% | Al₂O_{3,} |
| 14.6 | Gew.-% | Li₂O, |
| 2.9 | Gew.-% | Na₂O, |
| 4.9 | Gew.-% | TiO₂, |
| 1,1 | Gew.-% | ZrO₂, |
| 0,06 | Gew.-% | CaO und |
| 1.4 | Gew.-% | P₂O₅ |
enthält.

13. Ausgangsglas für Verblendkeramiken für dentale Restaurationen aus yttriumstabilisiertem Zirkoniumdioxid, **dadurch gekennzeichnet, dass** das Ausgangsglas
| | | |
|---|---|---|
| 70.5 | Gew.-% | SiO_{2,} |
| 4,9 | Gew.-% | Al₂O₃, |
| 14.8 | Gew.-% | Li₂O, |
| 3,0 | Gew.-% | Na₂O, |
| 5,0 | Gew.-% | TiO₂, |
| 1,1 | Gew.-% | ZrO₂, und |
| 0,7 | Gew.-% | CaF₂ |
enthält.

14. Verwendung einer Verblendkeramik gemäß einem der Ansprüche 1.oder 4 bis 10 oder eines Ausgangsglases gemäß einem der Ansprüche bis 7 oder 11 bis 13 zur Verblendung von dentalen Restaurationen aus yttriumstabilisiertem Zirkoniumdioxid.

15. Verfahren zur Verblendung von dentalen Restaurationen aus yttriumstabilisiertem Zirkoniumdioxid unter Verwendung einer Verblendkeramik gemäß einem der Ansprüche 1 oder 4 bis 10 oder eines Ausgangs-glases gemäß einem der Ansprüche 2 bis 7 oder 11 bis 13.

16. Verfahren zur Herstellung einer Verblendkeramik gemäß einem der Ansprüche 1 oder 4 bis 10, wobei eine Keimbildung im Temperaturbereich von 500 bis 680 °C und ein Verschmelzen und Kristallisieren im Temperaturbereich von. 800 bis 940 °C erfolgt.

17. Verfahren nach Anspruch 15 zur Verblendung von dentalen Restaurationen aus yttriumstabilisiertem Zirkoniumdioxid mit einer Verblendkaramik nach einem der Ansprüche 1 oder 4 bis 10, **gekennzeichnet durch** die folgenden Verfahrensschritte:
a) ein bei 1530 °C erschmolzenes Glas wird in Wasser gefrittet,
b) das gefrittete Glas wird zur Keimbildung bei einer Temperatur von 500 °C bis 680 °C und einem Zeitraum von zwei bis sechs Stunden getempert,
c) nach dem Abkühlen wird das getemperte Glas mechanisch pulverisiert,
d) unter Hinzufügen von Wasser wird das vorbehandelte Pulver in eine Paste überführt,
e) die Paste wird auf die Restaurationen aus yttriumstabilisiertem Zirkoniumdioxid aufgetragen und abschließend
f) einer Temperaturbehandlung von 800 °C bis 940 °C unterzogen, wobei in der Verblendkeramik als Hauptkristallphase Lithiumdisilicat auskristallisiert.

18. Verfahren nach Anspruch 17, **dadurch gekennzeichnet, dass** neben Lithiumdisilicat auch Lithiumalumosilicat sowie Lithium Titanium Oxid Silicat Li₂TiOSiO₄ auskristallisieren.

19. Verfahren nach Anspruch 17 oder 18, **dadurch gekennzeichnet, dass** dem gefritteten Glas ein kristalliner Zusatz aus gepulvertem Lithiumdisilicat, welches über eine Festkörperreaktion hergestellt wurde, zugefügt wird.

## Claims

1. Veneer ceramic for dental restorations made of yttrium-stabilized zirconium dioxide, **characterized in that** the veneer ceramic comprises the following components:
| | | |
|---|---|---|
| a) | SiO₂ | 58.0-74.0 % by weight |
| b) | Al₂O₃ | 4.0-19.0 % by weight |
| c) | Li₂O | 5.0-17.0 % by weight |
| d) | Na₂O | 4.0-12.0 % by weight |
| e) | ZrO₂ | 0.5-6.0 % by weight. |

2. Starting glass for veneer ceramics for dental restorations made of yttrium-stabilized zirconium dioxide, **characterized in that** the starting glass comprises the following components:
| | | |
|---|---|---|
| a) | SiO₂ | 58.0-74.0 % by weight |
| b) | Al₂O₃ | 4.0-19.0 % by weight |
| c) | Li₂O | 5.0-17.0 % by weight |
| d) | Na₂O | 4.0-12.0 % by weight |
| e) | ZrO₂ | 0.5-6.0 % by weight. |

3. Starting glass according to claim 2, **characterized in that** it further comprises powdered crystals, preferably powdered lithium disilicate.

4. Veneer ceramic according to claim 1 or starting glass according to claim 2 or 3, **characterized in that** in addition to ZrO₂ also 0.2 to 0.8 % by weight of TiO₂ are present.

5. Veneer ceramic according to claim 1 or 4 or starting glass according to any one of claims 2, 3 or 4 **characterized in that** the following components are present:
| | | |
|---|---|---|
| a) | SiO₂ | 58.0-72.0 % by weight |
| b) | Al₂O₃ | 4.0-18.0 % by weight |
| c) | Li₂O | 5.0-17.0 % by weight |
| d) | Na₂O | 4.0-11.0 % by weight |
| e) | ZrO₂ | 0.5- 5.5 % by weight |
| f) | TiO₂ | 0.2- 8.0 % by weight. |

6. Veneer ceramic according to any one of claims 1, 4 or 5 or starting glass according to any one of claims 2 to 5 **characterized in that** it comprises independently from one another the additives La₂O₃, B₂O₃, P₂O₅, CaO, MgO, ZnO and fluoride, with the concentration being in the range of up to 4.0 % by weight at most.

7. Veneer ceramic according to claim 6 or starting glass according to claim 6, **characterized in that** it comprises the following components:
| | | |
|---|---|---|
| a) | La₂O₃ | 1.0-4.0 % by weight |
| b) | B₂O₃ | 0.0-2.0 % by weight |
| c) | MgO | 0.0-2.0 % by weight |
| d) | CaO | 0.0-2.0 % by weight |
| e) | ZnO | 0.0-2.0 % by weight |
| f) | BaO | 0.0-1.0 % by weight |
| g) | P₂O₅ | 0.0-2.0 % by weight |
| h) | fluoride | 0.0-3.0 % by weight. |

8. Veneer ceramic according to any one of claims 1 or 4 to 7 or starting glass according to any one of claims 2 to 7, **characterized in that** it comprises oxides of coloring or fluorescing additions of the elements Ce, Fe, Mn, Sn, V, Cr, In, and of the rare earths Pr, Nd, Sm, Eu, Tb, Dy and Er singly or in combination.

9. Veneer ceramic according to claim 1 or 5, **characterized in that** it comprises other alkali oxides for the suppression of crystallization of beta-quartz mixed crystals.

10. Veneer ceramic according to any one of claims 1 or 4 to 9, **characterized in that** it comprises in addition to lithium disilicate also the crystal phases titanium oxide silicate Li₂TiOSiO₄, lithium alumosilicate (β-Spodumene) and smaller amounts of lithium metasilicate.

11. Starting glass for veneer ceramics for dental restorations made of yttrium-stabilized zirconium dioxide, **characterized in that** the starting glass comprises
| | |
|---|---|
| 71.1 % by weight | SiO₂, |
| 4.9 % by weight | Al₂O₃, |
| 14.9 % by weight | Li₂O, |
| 3.0 % by weight | Na₂O, |
| 5.0 % by weight | TiO₂ and |
| 1.1 % by weight | ZrO₂. |

12. Starting glass for veneer ceramics for dental restorations made of yttrium-stabilized zirconium dioxide, **characterized in that** the starting glass comprises
| | |
|---|---|
| 69.7 % by weight | SiO₂, |
| 4.8 % by weight | Al₂O₃, |
| 14.6 % by weight | Li₂O, |
| 2.9 % by weight | Na₂O, |
| 4.9 % by weight | TiO₂, |
| 1.1 % by weight | ZrO₂, |
| 0.6 % by weight | CaO, and |
| 1.4 % by weight | P₂O₅. |

13. Starting glass for veneer ceramics for dental restorations made of yttrium-stabilized zirconium dioxide, **characterized in that** the starting glass comprises
| | |
|---|---|
| 70.5 % by weight | SiO₂, |
| 4.9 % by weight | Al₂O₃, |
| 14.8 % by weight | Li₂O, |
| 3.0 % by weight | Na₂O, |
| 5.0 % by weight | TiO₂, |
| 1.1 % by weight | ZrO₂ and |
| 0.7 % by weight | CaF₂. |

14. Use of the veneer ceramic according to any one of claims 1 or 4 to 10 or of the starting glass according to any one of claims 2 to 7 or 11 to 13 for veneering dental restorations made of yttrium-stabilized zirconium dioxide.

15. Process for veneering dental restorations made of yttrium-stabilized zirconium dioxide by using the veneer ceramic according to any one of claims 1 or 4 to 10 or of the starting glass according to any one of claims 2 to 7 or 11 to 13.

16. Process for preparing a veneer ceramic according to any one of claims 1 or 4 to 10, wherein nucleation is carried out in the temperature range of 500 to 680°C and the melting and crystallization is carried out in the temperature range of 800 to 940°C.

17. Process according to claim 15 for veneering dental restorations made of yttrium-stabilized zirconium dioxide with the veneer ceramic according to any one of claims 1 or 4 to 10, **characterized by** the following steps:
a) a glass melted at 1530°C is fritted in water;
b) the fritted glass is tempered for nucleation at a temperature of 500°C to 680°C for 2 to 6 hours;
c) after cooling, the tempered glass is mechanically pulverized;
d) the pretreated powder is converted to a paste with the addition of water;
e) the paste is applied to the restorations made of yttrium-stabilized zirconium dioxide and
f) is then subjected to a heat treatment at 800°C to 940°C, whereby lithium disilicate is crystallized in the veneer ceramic as main crystal phase.

18. Process according to claim 17, **characterized in that** lithium aluminum silicate and lithium titanium oxide silicate Li₂TiOSiO₄ are also crystallized in addition to lithium disilicate.

19. Process according to claim 17 or 18, **characterized in that** a crystalline addition of powdered lithium disilicate which was produced by a solid state reaction is added to the fritted glass.

## Revendications

1. Céramique pour incrustations vestibulaires de restaurations dentaires en dioxyde de zirconium stabilisé à l'yttrium, **caractérisée en ce qu'**elle contient les constituants suivants :
| | | |
|---|---|---|
| a) | SiO₂ : | 58,0 - 74,0 % en poids |
| b) | Al₂O₃ : | 4,0 - 19,0% en poids |
| c) | Li₂O : | 5,0 - 17,0 % en poids |
| d) | Na₂O : | 4,0 - 12,0% en poids |
| e) | ZrO₂ : | 0,5 - 6,0% en poids. |

2. Verre de départ pour céramique pour incrustations vestibulaires de restaurations dentaires en dioxyde de zirconium stabilisé à l'yttrium, **caractérisé en ce que** le verre de départ contient les constituants suivants :
| | | |
|---|---|---|
| a) | SiO₂ : | 58,0 - 74,0 % en poids |
| b) | Al₂O₃ : | 4,0 - 19,0% en poids |
| c) | Li₂O : | 5,0 - 17,0 % en poids |
| d) | Na₂O : | 4,0 - 12,0% en poids |
| e) | ZrO₂ : | 0,5 - 6,0 % en poids. |

3. Verre de départ conforme à la revendication 2, **caractérisé en ce qu'**il contient en outre des cristaux pulvérisés, de préférence du disilicate de lithium pulvérisé.

4. Céramique pour incrustations vestibulaires, conforme à la revendication 1, ou verre de départ conforme à la revendication 2 ou 3, caractérisé(e) en ce qu'elle ou il contient en tant qu'autre formateur de germes, en plus de ZrO₂, 0,2 à 8,0 % en poids de TiO₂.

5. Céramique pour incrustations vestibulaires, conforme à la revendication 1 ou 4, ou verre de départ conforme à l'une des revendications 2, 3 et 4, caractérisé(e) en ce qu'elle ou il contient les constituants suivants :
| | | |
|---|---|---|
| a) | SiO₂ : | 58,0-72,0 % en poids |
| b) | Al₂O₃ : | 4,0-18,0 % en poids |
| c) | Li₂O : | 5,0 - 17,0 % en poids |
| d) | Na₂O : | 4,0-11,0% en poids |
| e) | ZrO₂ : | 0,5 - 5,5 % en poids |
| f) | TiO₂ : | 0,2-8,0% en poids. |

6. Céramique pour incrustations vestibulaires, conforme à l'une des revendications 1, 4 et 5, ou verre de départ conforme à l'une des revendications 2 à 5, caractérisé(e) en ce qu'elle ou il contient, indépendamment les uns des autres, des constituants additionnels La₂O₃, B₂O₃, P₂O₅, CaO, MgO, ZnO et fluorure, en une concentration maximale de 4,0 % en poids.

7. Céramique pour incrustations vestibulaires, conforme à la revendication 6, ou verre de départ conforme à la revendication 6, caractérisé(e) en ce qu'elle ou il contient les constituants suivants :
| | | |
|---|---|---|
| a) | La₂O₃: | 1,0 - 4,0 % en poids |
| b) | B₂O₃ : | 0,0 - 2,0 % en poids |
| c) | MgO : | 0,0 - 2,0% en poids |
| d) | CaO: | 0,0 - 2,0% en poids |
| e) | ZnO: | 0,0 - 2,0% en poids |
| f) | BaO: | 0,0 - 1,0% en poids |
| g) | P₂O₅ : | 0,0 - 2,0% en poids |
| h) | fluorure : | 0,0 - 3,0 % en poids. |

8. Céramique pour incrustations vestibulaires, conforme à l'une des revendications 1 et 4 à 7, ou verre de départ conforme à l'une des revendications 2 à 7, caractérisé(e) en ce qu'elle ou il contient, en tant que constituants additionnels colorants ou fluorescents, des oxydes des éléments Ce, Fe, Mn, Sn, V, Cr et In, ainsi que des terres rares Pr, Nd, Sm, Eu, Tb, Dy et Er, seuls ou en combinaison.

9. Céramique pour incrustations vestibulaires, conforme à la revendication 1 ou 5, **caractérisée en ce qu'**elle contient d'autres oxydes alcalins, pour abaisser la pression de cristallisation des cristaux mixtes de quartz haute température.

10. Céramique pour incrustations vestibulaires, conforme à l'une des revendications 1 et 4 à 9, **caractérisée en ce qu'**elle contient, outre du disilicate de lithium, des phases cristallines silicate-oxyde de lithium et titane (Li₂TiOSiO₄) et alumino-silicate de lithium (β-spodumène), et de faibles quantités de métasilicate de lithium.

11. Verre de départ pour céramique pour incrustations vestibulaires de restaurations dentaires en dioxyde de zirconium stabilisé à l'yttrium, **caractérisé en ce que** le verre de départ contient les constituants suivants :
71,1 % en poids de SiO₂
4,9 % en poids de Al₂O₃
14,9 % en poids de Li₂O
3,0 % en poids de Na₂O
5,0 % en poids de TiO₂
et 1,1 % en poids de ZrO₂.

12. Verre de départ pour céramique pour incrustations vestibulaires de restaurations dentaires en dioxyde de zirconium stabilisé à l'yttrium, **caractérisé en ce que** le verre de départ contient les constituants suivants :
69,7 % en poids de SiO₂
4,8 % en poids de Al₂O₃
14,6 % en poids de Li₂O
2,9 % en poids de Na₂O
4,9 % en poids de TiO₂
1,1 % en poids de ZrO₂
0,6 % en poids de CaO
et 1,4 % en poids de P₂O₅.

13. Verre de départ pour céramique pour incrustations vestibulaires de restaurations dentaires en dioxyde de zirconium stabilisé à l'yttrium, **caractérisé en ce que** le verre de départ contient les constituants suivants :
70,5 % en poids de SiO₂
4,9 % en poids de Al₂O₃
14,8 % en poids de Li₂O
3,0 % en poids de Na₂O
5,0 % en poids de TiO₂
1,1 % en poids de ZrO₂
et 0,7 % en poids de CaF₂.

14. Utilisation d'une céramique pour incrustations vestibulaires conforme à l'une des revendications 1 et 4 à 10, ou d'un verre de départ conforme à l'une des revendications 2 à 7 et 11 à 13, pour la réalisation d'incrustations vestibulaires sur des restaurations dentaires en dioxyde de zirconium stabilisé à l'yttrium.

15. Procédé de réalisation d'incrustations vestibulaires sur des restaurations dentaires en dioxyde de zirconium stabilisé à l'yttrium, avec utilisation d'une céramique pour incrustations vestibulaires conforme à l'une des revendications 1 et 4 à 10, ou d'un verre de départ conforme à l'une des revendications 2 à 7 et 11 à 13.

16. Procédé de préparation d'une céramique pour incrustations vestibulaires conforme à l'une des revendications 1 et 4 à 10, dans lequel ont lieu une étape de formation de germes dans l'intervalle de température allant de 500 à 680°C, et une étape de fusion et cristallisation dans l'intervalle de température allant de 800 à 940°C.

17. Procédé, conforme à la revendication 15, de réalisation d'incrustations vestibulaires sur des restaurations dentaires en dioxyde de zirconium stabilisé à l'yttrium, au moyen d'une céramique pour incrustations vestibulaires conforme à l'une des revendications 1 et 4 à 10, **caractérisé par** les étapes de procédé suivantes :
a) on fritte dans de l'eau un verre fondu à 1530 °C;
b) on attrempe le verre fritté à une température de 500 à 680 °C et durant un laps de temps de deux à six heures, pour qu'il se forme des germes ;
c) après avoir fait refroidir le verre attrempé, on le pulvérise par voie mécanique ;
d) par addition d'eau, on transforme la poudre traitée ainsi obtenue en une pâte;
e) on dépose cette pâte sur les restaurations en dioxyde de zirconium stabilisé à l'yttrium ;
f) et l'on soumet enfin le tout à un traitement thermique à une température de 800 à 940°C, grâce à quoi du disilicate de lithium cristallise en tant que principale phase cristalline dans la céramique pour incrustations vestibulaires.

18. Procédé conforme à la revendication 17, **caractérisé en ce que**, outre du disilicate de lithium, de l'alumino-silicate de lithium et du silicate-oxyde de lithium et titane (Li₂TiOSiO₄) cristallisent aussi.

19. Procédé conforme à la revendication 17 ou 18, **caractérisé en ce que** l'on ajoute au verre fritté un surplus cristallin de disilicate de lithium pulvérisé qui a été préparé par réaction en phase solide.
